# EUROPEAN PATENT APPLICATION

(11) **EP 3 968 060 A1**
(43) Date of publication of application: **16.03.2022**
(21) Application number: 20196293.3
(22) Date of filing: 15.09.2020
(51) Int. Cl.: G02B 1/111, A41D 13/11, A41D 13/12, A61K 33/00

(54) **ANTI-REFLECTIVE AND ANTI-VIRAL PROTECTIVE SHIELD, METHOD FOR MANUFACTURING AN ANTI-REFLECTIVE AND ANTI-VIRAL PROTECTIVE SHIELD AND/OR AN ANTI-REFLECTIVE AND ANTI-VIRAL VISOR FOR A FACE PROTECTION SYSTEM, AND ANTI-REFLECTIVE AND ANTI-VIRAL VISOR FOR A FACE PROTECTION SYSTEM**

(71) Applicant: Applied Materials, Inc., Santa Clara, CA 95054 (US)
(72) Inventor: Deppisch, Thomas, 63743 Aschaffenburg (DE); Haberkorn, Edgar, 63637 Jossgrund (DE); Nick, Christian, 63743 Aschaffenburg (DE); Pieralisi, Fabio, 63799 Aschaffenburg (DE)
(74) Representative: Zimmermann & Partner Patentanwälte mbB

(57) **Abstract**

An anti-reflective and anti-viral protective shield, an anti-reflective and anti-viral visor for a face protection system, a method for manufacturing an anti-reflective and anti-viral protective shield and/or an anti-reflective and anti-viral visor for a face protection system, and a face protection system comprising an anti-reflective and anti-viral visor are provided. The anti-reflective and anti-viral protective shield and the anti-reflective and anti-viral visor for a face protection system include a layer stack including a first layer, a second layer, a third layer, and a fourth layer arranged in this order. The first layer and the third layer have a refractive index of at least 1.9, and the second layer and the fourth layer have a refractive index of less than 1.7. Further, the anti-reflective and anti-viral protective shield and the anti-reflective and anti-viral visor for a face protection system also includes an upper layer over the layer stack. The upper layer includes at least one material selected from the group consisting of TiO₂, ZnO, CuO, Ag-TiO₂, and combinations thereof.

## Description

### TECHNICAL FIELD

Embodiments of the present disclosure relate to an anti-reflective and anti-viral protective shield, a method for manufacturing an anti-reflective and anti-viral protective shield and/or an anti-reflective and anti-viral visor for a face protection system, and an anti-reflective and anti-viral visor for a face protection system.

### BACKGROUND ART

Presently the whole world is facing a pandemic and a global crisis caused by a novel virus that was discovered to be the cause of a large and rapidly spreading outbreak of respiratory disease, including potentially fatal pneumonia. The virus has been provisionally designated 2019-nCoV and later given the official name SARS-CoV-2. The disease caused by the virus was officially named Coronavirus Disease 2019 (COVID-19) by the World Health Organization.

With the current COVID-19 outbreak, the need for effective personal protection from COVID-19 and/or any other virus or bacteria has become more relevant than ever. In this regard, examples of personal protection devices commonly used include visors, which are commonly used in face shields, and protective shields, such as freestanding shields for desks or counters and visors. In particular, freestanding shields for counters are currently used in stores to protect retail workers from infectious droplets from customer sneezes and coughs. Further, visors, which are commonly employed in face protection systems, are usually used by many workers (e.g., medical, dental, veterinary) and the public in general for protection of the facial area and associated mucous membranes (eyes, nose, mouth) from splashes, sprays, and spatter of body fluids.

Although the above-mentioned visors and protective shields provide certain personal protection, there is still a need for an improved visor and a protective shield.

### SUMMARY

In light of the above, an anti-reflective and anti-viral protective shield, an anti-reflective and anti-viral visor for a face protection system, a method for manufacturing an anti-reflective and anti-viral protective shield and/or an anti-reflective and anti-viral visor for a face protection system, and a face protection system comprising an anti-reflective and anti-viral visor are provided. Further aspects, advantages, and features of the present disclosure are apparent from the claims, the description, and the accompanying drawings.

According to an aspect of the present disclosure, an anti-reflective and anti-viral protective shield is provided. The anti-reflective and anti-viral protective shield includes a substrate, a layer stack over the substrate, and an upper layer over the layer stack. The layer stack comprises at least a first layer, a second layer, a third layer, and a fourth layer arranged in this order. The first layer and the third layer have a refractive index of at least 1.9, and the second layer and the fourth layer have a refractive index of less than 1.7. The upper layer comprises at least one material selected from the group consisting of TiO₂, ZnO, CuO, Ag-TiO₂, and combinations thereof.

According to an additional aspect of the present disclosure, an anti-reflective and anti-viral visor for a face protection system is provided. The anti-reflective and anti-viral visor for a face protection system includes a substrate, a layer stack over the substrate, and an upper layer over the layer stack. The layer stack comprises at least a first layer, a second layer, a third layer, and a fourth layer arranged in this order. The first layer and the third layer have a refractive index of at least 1.9, and the second layer and the fourth layer have a refractive index of less than 1.7. The upper layer comprises at least one material selected from the group consisting of TiO₂, ZnO, CuO, Ag-TiO₂, and combinations thereof.

According to another aspect of the present disclosure, a method for manufacturing an anti-reflective and anti-viral protective shield and/or an anti-reflective and anti-viral visor for a face protection system is provided. The method includes: alternately depositing a first layer material and a second layer material on a substrate to form at least four layers and depositing a third layer material on the layer stack to form an upper layer. The first layer and the third layer have a refractive index of at least 1.9, and the second layer and the fourth layer have a refractive index of less than 1.7. The upper layer comprises at least one material selected from the group consisting of TiO₂, ZnO, CuO, Ag-TiO₂, and combinations thereof.

### BRIEF DESCRIPTION OF THE DRAWINGS

So that the manner in which the above recited features of the present disclosure can be understood in detail, a more particular description of the disclosure, briefly summarized above, may be had by reference to embodiments. The accompanying drawings relate to embodiments of the disclosure and are described in the following:
Figures 1A-C show cross-sectional views of an anti-reflective and anti-viral protective shield and/or an anti-reflective and anti-viral visor for a face protection system according to embodiments described herein;
Figures 2A and 2B show cross-sectional views of an anti-reflective and anti-viral protective shield and/or an anti-reflective and anti-viral visor for a face protection system according to embodiments described herein;
Figure 3 shows a graph of a reflectance of an anti-reflective and anti-viral protective shield and/or an anti-reflective and anti-viral visor for a face protection system according to embodiments described herein;
Figure 4 shows a face protection system according to embodiments described herein.
Figure 5 shows a schematic view of a deposition apparatus used for manufacturing an anti-reflective and anti-viral protective shield and/or an anti-reflective and anti-viral visor for a face protection system according to embodiments described herein; and
Figure 6 shows a flow chart of a method for manufacturing an anti-reflective and anti-viral protective shield and/or an anti-reflective and anti-viral visor for a face protection system according to embodiments described herein.

### DETAILED DESCRIPTION OF EMBODIMENTS

Reference will now be made in detail to the various embodiments of the disclosure, one or more examples of which are illustrated in the figures. Within the following description of the drawings, the same reference numbers refer to same components. Generally, only the differences with respect to individual embodiments are described. Each example is provided by way of explanation of the disclosure and is not meant as a limitation of the disclosure. Further, features illustrated or described as part of one embodiment can be used on or in conjunction with other embodiments to yield yet a further embodiment. It is intended that the description includes such modifications and variations.

The need for effective personal protection from COVID-19 and/or any other virus or bacteria has become more relevant nowadays than ever. Protective shields are most commonly used for this purpose in stores to protect retail workers from infectious droplets from customer sneezes and coughs. Further, visors are used, for instance, in face shields by many workers (e.g., medical, dental, veterinary) and the public in general for protection of the facial area. Face shields are preferred in comparison to face masks as effective personal protection since breathing with a face mask requires more effort.

While the above-mentioned visors and protective shields provide a great deal of personal protection, a problem related to the use of such visors and protective shields is that the visors and protective shields also produce a glare from bright lights illuminating an operation room or a store, which can impair the vision of the medical professional in the operation room or workers in the store. Accordingly, the glare produced by the visors and protective shields obstruct the vision within the field of view, which may cause distractions away from the immediate task at hand.

Further, in order to continuously use such visors and protective shields without substituting for a clean, new visor or protective shield, the visors or protective shields have to be wiped constantly and an anti-viral and/or anti-microbial solution has to be applied, which increase the time, work, and cost of maintenance of such visors and protective shields to prevent the spread of COVID-19 through touch.

The present disclosure overcomes these drawbacks by providing a protective shield and a visor for a face protection system having combined anti-reflection and anti-viral properties and a method for manufacturing the anti-reflective and anti-viral protective shield and the anti-reflective and anti-viral visor.

Before various embodiments of the present disclosure are described in more detail, some aspects with respect to some terms and expressions used herein are explained.

In the present disclosure, "protective shield" may be understood as a piece of material used for personal protection from splashes, sprays, and spatter of body fluids coming from an external individuum, and designed to cover at least a facial area of an individuum. The term "protective shield" may be also understood as a material designed to be attached to a desk, a counter or any other article that is used to make a room or building suitable for living or working and/or a ceiling, a floor or any part of a room. Further, the term "visor" may be understood as a material designed to cover a face area of an individuum, particularly eyes, nose, and mouth, and more particularly eyes, being attached to a headwear or headgear and used for personal protection from splashes, sprays, and spatter of body fluids coming from an external individuum.

The term "anti-viral" may be understood as a property to destroy a virus or a property to suppress the ability of a virus to replicate and, hence, inhibit the capability of the virus to multiply and reproduce. The term "refractive index" (or index of refraction) n of a material or optical medium is a dimensionless number that describes how light, or any other radiation, propagates through that material. It is defined as n=c/v, where c is the speed of light in vacuum and v is the speed of light in the material.

The anti-reflective and anti-viral protective shield and/or the anti-reflective and anti-viral visor according to embodiments described herein can have color neutrality. The color neutrality provides improved optical characteristics of the anti-reflective and anti-viral protective shield and/or the anti-reflective and anti-viral visor.

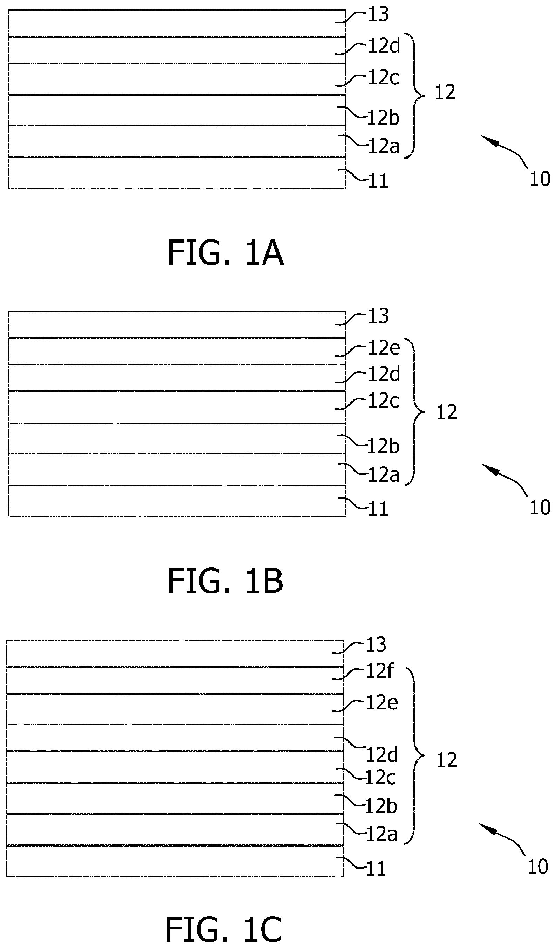

Figures 1A-C show cross-sectional views of an anti-reflective and anti-viral protective shield 10 and/or an anti-reflective and anti-viral visor 10 for a face protection system according to embodiments described herein. Although various embodiments of the present disclosure are described with reference to the anti-reflective and anti-viral protective shield 10 for the sake of simplicity, the various embodiments referring to the anti-reflective and anti-viral protective shield 10 of the present disclosure may also apply or refer to the anti-reflective and anti-viral visor 10 for the face protection system of the present disclosure.

According to some embodiments, the anti-reflective and anti-viral protective shield 10 includes a substrate 11, particularly a transparent substrate. The term "substrate" as used herein can particularly embrace flexible substrates such as a web or a foil. However, the present disclosure is not limited thereto, and the term "substrate" may also embrace inflexible substrates, e.g., slices of transparent crystal such as sapphire or the like, or a glass plate.

In some embodiments, the substrate 11 may comprise at least one material selected from the group consisting of polycarbonate, cellulose acetate propionate, polyvinyl chloride, polyacrylate and/or derivatives of polyacrylate, polymethacrylate and/or derivatives of polymethacrylate, polyethylene terephthalate glycol, polyethylene terephthalate, and combinations thereof.

The term "transparent" as used herein can particularly include the capability of a structure to transmit light with relatively low scattering, so that, for example, light transmitted therethrough can be seen in a substantially clearly manner.

Further, according to some embodiments, the anti-reflective and anti-viral protective shield 10 includes a layer stack 12 over the substrate 11. Furthermore, the anti-reflective and anti-viral protective shield 10 includes an upper layer 13 over the layer stack 12.

According to some embodiments, the upper layer 13 may include at least one material selected from the group consisting of TiO₂, ZnO, CuO, Ag-TiO₂, and combinations thereof.

The upper layer 13 may impair the anti-reflective properties of the layer stack 12 as described in the present disclosure due to the fact that materials having anti-viral properties may worsen the anti-reflective properties of the layer stack 12. Therefore, in some embodiments, the upper layer 13 has a thickness equal to or smaller than 5 nm, particularly a thickness equal to or smaller than 3 nm, and more particularly a thickness equal to or smaller than 1 nm. This is advantageous because it could be shown that the maximum thickness of the upper layer of below 5 nm, particularly below 3 nm, or even below 1 nm allows to maintain the anti-reflective properties of the layer stack 12 to a satisfying extent. Consequently, the protective shield and/or the visor for the face protection system show both properties, namely anti-reflective and anti-viral properties.

In some embodiments, the upper layer, in particular if the upper layer has a size as described in the previous paragraph, can be disposed or deposited directly on the layer stack 12. This provides enhanced anti-reflective properties.

An aspect of the present disclosure is to provide a protective shield 10 and/or a visor 10 having anti-reflective and anti-viral properties, particularly to provide a protective shield 10 and/or a visor 10 with an upper layer 13 having anti-viral properties and a layer stack 12 having anti-reflective properties. Particularly, the material of the upper layer 13 has anti-viral properties.

Further, the thickness and/or material of the upper layer 13 allows that the thicknesses and/or the materials of the layers of the layer stack 12 improve a light transmittance of a substrate, particularly the thicknesses of the layers of the layer stack 12 are designed to provide a higher light transmittance of a substrate than a light transmittance of an (uncoated) substrate on which the layer stack is disposed or deposited. Particularly, an aspect of the present disclosure is to increase a light transmittance of a substrate in the visible region. The thickness of the upper layer 13 favors the anti-reflection properties of the stack layer 12, since the anti-reflection functionality of the stack layer 12 stays unaffected.

According to some embodiments, the layer stack 12 over the substrate 11 of the present embodiments can consist of a number of layers formed (e.g., by deposition) one on top of another. In some embodiments, the layer stack 12 can be directly disposed or deposited on the substrate 11. In some embodiments, the layers of the layer stack 12 can be directly disposed or deposited on each other.

In some embodiments, at least one of the layers of the layer stack 12 may have a thickness of more than 10 nm, particularly a thickness of more than 20 nm, and more particularly a thickness of more than 30 nm. In some embodiments, at least one of the layers of the layer stack 12 may have a thickness in the range of 30 nm and 400 nm, particularly in the range of 70 nm to 300 nm, and more particularly in the range of 100 nm to 150 nm. Thicknesses and/or optical properties of the individual layers of the layer stack 12 can be different.

The layer stack 12 of the present disclosure may include alternating layers, particularly alternating layers comprising different materials. The layers of the layer stack 12 may include a low refractive index material and a high refractive index material in a successive manner.

In some embodiments, at least one of the layers of the layer stack 12 can be dielectric layers. According to some embodiments, which can be combined with other embodiments described herein, the first layer 12a, the second layer 12b, the third layer 12c, and the fourth layer 12d are dielectric layers. In some embodiments, all of the layers of the layer stack 12 are dielectric layers.

According to some embodiments, which can be combined with other embodiments described herein, the uppermost layer of the layer stack 12, i.e., the last layer arranged e.g. over the substrate and being part of the layer stack 12 such as the fourth layer 12d, the fifth layer 12e, or the sixth layer 12f shown in figures 1A to 1C, respectively, may have a low refractive index. The layer stack 12 having the uppermost layer with the low refractive index provides improved optical characteristics. In some embodiments, the at least one material of the upper layer 13 may differ from the material of the uppermost layer of the layer stack 12. In some embodiments, the upper layer 13 can be directly disposed or deposited on the uppermost layer of the layer stack 12.

The present disclosure provides a layer stack 12 with anti-reflection properties. According to some embodiments, a layer stack 12 with four layers may be referred to as NONO, a layer stack 12 with five layers may be referred to as NONON and a layer stack 12 with six layers may be referred to as NONONO.

The symbols N and O may denote a material of the layers. In some embodiments, the symbol N denotes the material or layer with high refractive index (e.g., SiNx) and the symbol O denotes the material or layer with low refractive index (e.g., SiOx). The low refractive index can be in the range of 1.4 to 1.6, specifically about n=1.46. However, it is to be understood that the present disclosure is not limited to SiNx and SiOx, and that any suitable materials having the high refractive index of at least 1.9 and the low refractive index of less than 1.7 could be used for the layers with the high refractive index and the layers with the low refractive index, respectively.

Some examples can be insulating materials with high and low refractive indexes, for example SiOx, TiOx, NbOx, SiNx, SiOxNy, AlOx, AlOxNy, TaOx, an organic material such as a polymer material, and combinations thereof.

In some embodiments, an extinction coefficient of the materials with the high refractive index and the low refractive index can be small. The index of refraction and the extinction coefficient are the real part and the imaginary part, respectively, of the complex index of refraction. Particularly, when light passes through a medium, some part of the light will be absorbed. This can be described by defining the complex index of refraction as being equal to n+ik. The real part "n" indicates the phase velocity, while the imaginary part "ik" indicates the amount of absorption loss when the electromagnetic wave propagates through the material.

According to some embodiments, with an optimized layer stack 12, the transmittance of a NONO/NONONO design can be enhanced above the transmittance for instance of uncoated PET. In comparison to non-optimized NONON/NONONON designs, the (absolute) transmittance gain can be in the range of about 4% to about 6% (e.g., increasing a transmittance (Ty) from about 88% to about 92-94%). The contrast/color difference can be b*<0.3 (b* value as defined by the International Commission on Illumination (CIE) in 1976).

In figure 1A, a cross-sectional view of an anti-reflective and anti-viral protective shield 10 and/or an anti-reflective and anti-viral visor 10 for a face protection system are depicted. Particularly, a substrate 11, a layer stack 12, and an upper layer 13 according to embodiments of the present disclosure are depicted in figure 1A. The layer stack 12 includes at least a first layer 12a, a second layer 12b, a third layer 12c, and a fourth layer 12d arranged in this order. The first layer 12a and the third layer 12c have a refractive index of at least 1.9, and the second layer 12b and the fourth layer 12d have a refractive index of less than 1.7.

As an example, the first layer 12a and the third layer 12c may include or be made of SiNx, and the second layer 12b and the fourth layer 12d may include or be made of SiO₂.

The first layer 12a and the third layer 12c having a refractive index of at least 1.9 and the second layer 12b and the fourth layer 12d having a refractive index of less than 1.7 provide a layer stack 12 having an anti-reflection function and, together with the upper layer 13, provide combined anti-reflection and anti-viral functions. As an example, a light transmittance of the layer stack 12 can be about 94%, particularly in a wavelength range of about 400 nm to 700 nm.

According to some embodiments, which can be combined with other embodiments herein, the layer stack 12 can include one or more further layers over the fourth layer 12d, particularly a fifth layer 12e or a fifth layer 12e and a sixth layer 12f as shown in figures 1B and 1C, respectively.

In figure 1B, a layer stack 12 according to further embodiments of the present disclosure is depicted. The layer stack 12 of figure 1B is similar to the layer stack 12 of figure 1A with the difference being that a fifth layer 12e is arranged over the fourth layer 12d. In some implementations, the first layer 12a, the third layer 12c, and the fifth layer 12e may include or be made of SiNx, and the second layer 12b and the fourth layer 12d may include or be made of SiO₂. A light transmittance of the layer stack 12 can be about 88% to about 92%, particularly in a wavelength range of about 400 nm to 700 nm.

In figure 1C, a layer stack 12 according to further embodiments of the present disclosure is depicted. The layer stack 12 is similar to the layer stack 12 of figure 1B, with the difference being that a sixth layer 12f is arranged over the fifth layer 12e. In some embodiments, the first layer 12a, the third layer 12c and the fifth layer 12e may include or be made of SiNx, and the second layer 12b, the fourth layer 12d and the sixth layer 12f may include or be made of SiO₂. A transmittance of the layer stack can be about 94%, particularly in a wavelength range of about 400 nm to 700 nm.

In some implementations, the odd numbered layers of the layer stack 12 have the refractive index of at least 1.9, and the even numbered layers of the layer stack 12 have the refractive index of less than 1.7. The terms "odd" and "even" as used throughout this application refer to parity in mathematics, i.e. that an integer is even if it is evenly divisible by two and odd if it is not even. As an example, the odd numbered layers of the layer stack 12 can be the first, third, fifth etc. layers, and the even numbered layers of the layer stack 12 can be the second, fourth, sixth, etc. layers.

The odd numbered layers of the layer stack 12 having a refractive index of at least 1.9 and the even numbered layers of the layer stack 12 having a refractive index of less than 1.7 provide a layer stack 12 having an anti-reflection function.

The first layer to the sixth layer of the layer stack 12, the odd numbered layers of the layer stack 12 and the even numbered layers of the layer stack 12 as referred to in this application are the layers of the layer stack 12 providing the anti-reflection functions, i.e., the layers of the layer stack 12 having the refractive index of at least 1.9 or the refractive index of less than 1.7. Thus, the numbering excludes any other layers that could additionally be provided, such as seed layers, hard coatings, adhesive layers and the like.

A light transmittance of the layer stack 12 and/or a light transmittance of the layer stack 12 and the upper layer 13 can be at least 85%, and particularly more than 90%. As an example, the layer stack 12 and/or the layer stack 12 and the upper layer 13 may have a light transmittance in the range of 87 to 95%, specifically 88% or 89%, and more specifically 93% or 94%. Similarly, as an example, the layer stack 12 including the substrate 11 and/or the layer stack 12 and the upper layer 13 including the substrate 11 may have a light transmittance in the range of 87 to 95%, specifically 88% or 89%, and more specifically 93% or 94%. The anti-reflection function of the layer stack 12 is insusceptible to the material and/or the thickness of the upper layer 13 as described in the present disclosure.

As an example, the odd numbered layers of the layer stack 12 can have a refractive index of about at least 1.9, particularly of about 2. The even numbered layers of the layer stack 12 can have a refractive index of less than 1.7, particularly a refractive index of less than 1.5, and more particularly of about 1.46. According to some embodiments, the odd numbered layers of the layer stack 12 include at least one of SiNx, NbOx, SiN, SiOxNy, AlOx, AlOxNy, TiOx TaOx, an organic material such as a polymer material, and/or combinations thereof, and/or the even numbered layers of the layer stack 12 include at least one of SiOx, MgFx, SiOxNy, an organic material such as a polymer material, and/or combinations thereof.

As an example, the first layer 12a, e.g. a first dielectric layer, can have a high refractive index. By successively providing layers with alternating or different refractive indexes, a layer stack 12 having anti-reflection characteristics can be provided. According to some embodiments, which can be combined with other embodiments described herein, layers of the layer stack 12 having the lower refractive indexes (e.g. the even numbered layers) can be provided by depositing layers containing SiOx, MgFx, SiOxNy, an organic material such as a polymer material, and/or combinations thereof, or the like. For example, layers of the layer stack 12 having the higher refractive indexes (e.g. the odd numbered layers) can be provided by depositing layers containing NbOx, SiNx, SiN, SiOxNy, AlOx, AlOxNy, TiOx TaOx, an organic material such as a polymer material, and/or combinations thereof, or the like.

According to some implementations, the layers of the layer stack 12 and/or the upper layer 13 (e.g., dielectric films) can be manufactured by chemical vapor deposition or physical vapor deposition, for example sputtering or evaporation. Some examples can be insulating materials with high and low refractive indexes, for example SiOx, SiN, TiOx, NbOx, SiNx, SiOxNy, AlOx, AlOxNy, TaOx, an organic material such as a polymer material, and/or combinations thereof and/or TiO₂, ZnO, CuO, Ag-TiO₂, and combinations thereof.

According to some embodiments, which can be combined with other embodiments herein, the thickness of each of the odd numbered layers of the layer stack 12 can be less than the thickness of each of the even numbered layers of the layer stack 12.

According to some embodiments, which can be combined with other embodiments herein, the layers of the layer stack 12 can be formed or arranged over each other. In the examples of figures 1A-C, the second layer 12b is formed or arranged over the first layer 12a, the third layer 12c is formed or arranged over the second layer 12b and the fourth layer 12d is formed or arranged over the third layer 12c. In other words, in some embodiments, no further layers or films are present between the layers of the layer stack. In some other embodiments, further layers can be provided between at least some of the layers of the layer stack.

As an example, when reference is made to the term "over", i.e. one layer being over the other, it is understood that, starting from the first layer 12a, the second layer 12b is deposited over the first layer 12a, and a further layer, deposited after the second layer 12b, is thus over the second layer 12b and over the first layer 12a. In other words, the term "over" is used to define an order of layers, layer stacks, and/or films wherein the starting point is the substrate, which is irrespective of whether the layer stack 12 is depicted upside down or not.

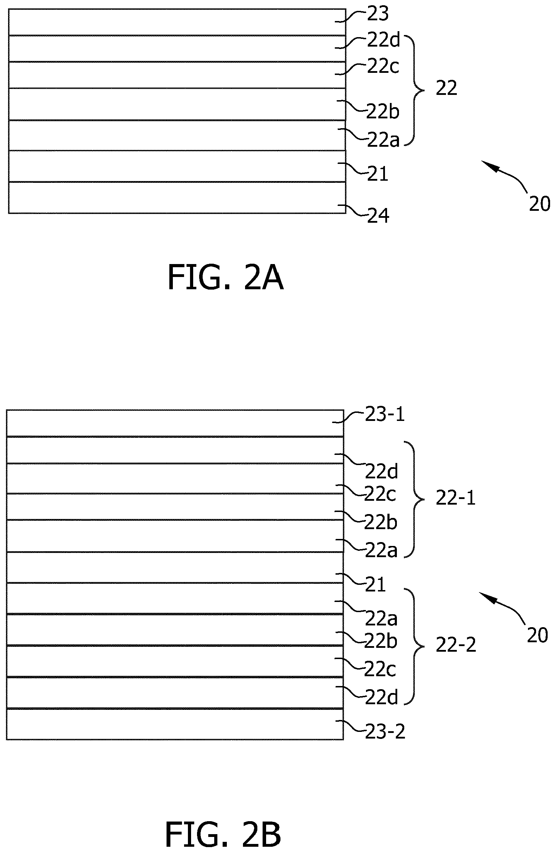

Figures 2A and 2B show cross-sectional views of an anti-reflective and anti-viral protective shield 20 and/or an anti-reflective and anti-viral visor 20 for a face protection system according to embodiments described herein. According to some embodiments, the anti-reflective and anti-viral protective shield 20 and/or an anti-reflective and anti-viral visor 20 for a face protection system includes a substrate 21, particularly a transparent substrate. The substrate 21 includes a first surface side and a second surface side. The first surface side of the substrate is opposite the second side of the substrate.

Particularly, in figure 2A, the anti-reflective and anti-viral protective shield 20 and/or the anti-reflective and anti-viral visor 20 for a face protection system includes a layer stack 22 over the first surface side of the substrate 21. As an example, the layer stack 22 may include a first layer 22a, a second layer 22b, a third layer 22c, and a fourth layer 22d. Thicknesses, materials and/or other properties of the layer stack 22, layers of the layer stack 22 and/or substrate 21 are those described for any other embodiment of the present disclosure. Further, the anti-reflective and anti-viral protective shield 20 and/or the anti-reflective and anti-viral visor 20 for a face protection system includes an upper layer 23 over the layer stack 22. In some embodiments, the upper layer 23 can be directly deposited over the layer stack 22.

Further, the anti-reflective and anti-viral protective shield 20 and/or the anti-reflective and anti-viral visor 20 for a face protection system includes an anti-viral layer 24 over the second surface side of the substrate 21. In some embodiments, the anti-viral layer 24 can be directly deposited over the second surface side of the substrate 21. The thickness and/or material of the anti-viral layer are similar to the thickness and/or material of the upper layer 23 as described of the present disclosure.

In figure 2B, the anti-reflective and anti-viral protective shield 20 and/or the anti-reflective and anti-viral visor 20 for a face protection system includes a first layer stack 22-1 over the first surface side of the substrate 21 and a second layer stack 22-2 over the second surface side of the substrate 21. As an example, the first layer stack 22-1 and/or the second layer stack 22-2 may include a first layer 22a, a second layer 22b, a third layer 22c, and a fourth layer 22d. Further, the anti-reflective and anti-viral protective shield 20 and/or the anti-reflective and anti-viral visor 20 for a face protection system includes a first upper layer 23-1 over the first layer stack 22-1 and a second upper layer 23-2 over the second layer stack 22-2.

In some embodiments, the first upper layer 23-1 and/or the second upper layer 23-2 can be directly deposited over the first layer stack 22-1 and/or the second layer stack 22-2, respectively. Thicknesses, materials and/or other properties of the first layer stack 22-1, the second layer stack 22-2, the first upper layer 23-1, the second upper layer 23-2 and/or the substrate 21 are those described for any other embodiment of the present disclosure. Thicknesses, materials and/or other properties of the first layer stack 22-1 can be different or similar to thicknesses, materials and/or other properties of the second layer stack 22-2.

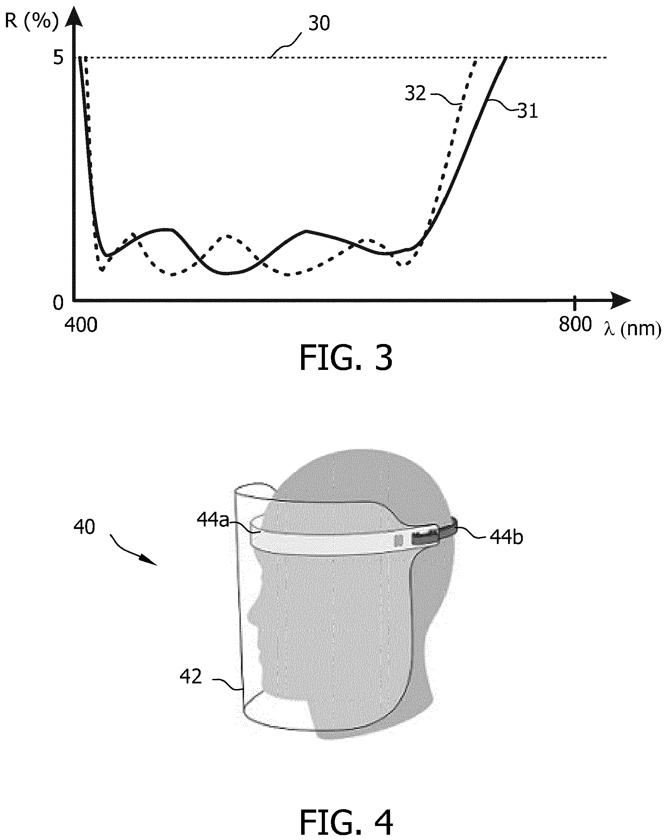

Figure 3 shows a graph of a reflectance of layer stacks according to embodiments described herein. The term "reflectance" as used herein is the fraction of incident electromagnetic power that is reflected. The term "reflectance" may be synonymously used with the term "reflectivity".

In figure 3, the γ-axis of the graph denotes a reflectance in % (percent), and the x-axis denotes a wavelength λ (lambda) in units of nanometer (nm). Denoted with reference numeral 30 is a reflectance of an uncoated PET substrate (about 5%). Denoted with reference numeral 31 is a reflectance of a layer stack according to embodiments described herein with four layers (NONO), which is less than 1 percent in a range from about 420 nm to about 680 nm. Reference numeral 32 indicates a reflectance of a layer stack according to embodiments described herein with six layers (NONONO), which is also less than 1 percent in a range from about 420 nm to about 680 nm.

Figure 4 shows a face protection system 40 according to embodiments described herein. The face protection system 40 includes an anti-reflective and anti-viral visor 42 according to embodiments described in the present disclosure. Further, the face protection system 40 may include a head connection unit such as a headwear or a headgear. Examples of headwear and headgear are a hat, a helmet or any other covering for the head. As an example, the face protection system 40 may include a forehead pad 44a and a head-engaging band 44b. The forehead pad 44a may be suitable to be positioned upon a wearer's forehead. The head-engaging band 44b may be suitable to maintain the position of the face protection system 40 upon the wearer's head. The forehead pad 44a may include a foam.

Methods for depositing a material for instance on a substrate may include a physical vapor deposition (PVD) process, a chemical vapor deposition (CVD) process, a plasma enhanced chemical vapor deposition (PECVD) process, etc. As an example, the process is performed in a process apparatus or process chamber, where the substrate to be coated is located. A deposition material is provided in the apparatus. A plurality of materials such as oxides, nitrides or carbides thereof may be used for deposition. Further, other processes like etching, structuring, annealing, or the like can be conducted in processing chambers.

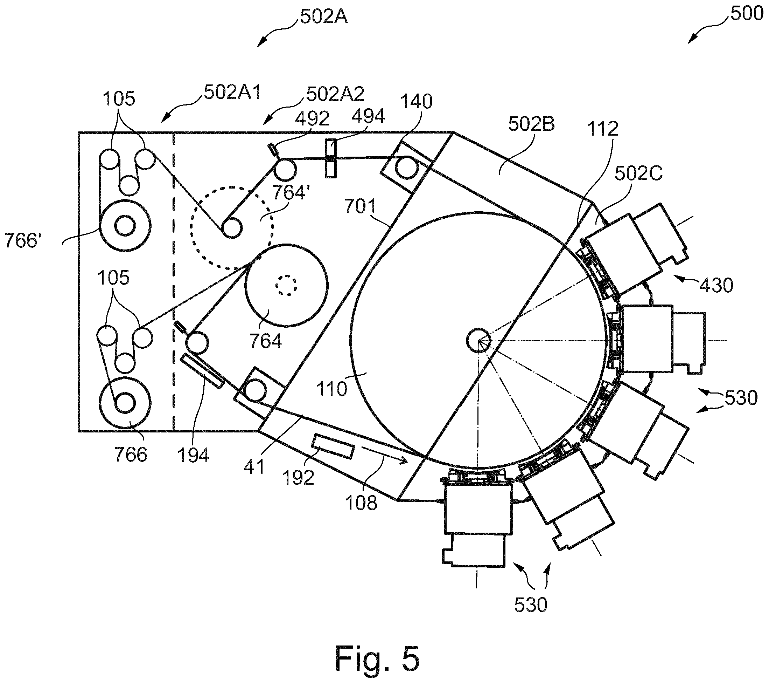

Figure 5 shows a schematic view of an apparatus 500, e.g. a roll-to-roll deposition apparatus, for depositing or coating the layers according to embodiments described herein, and particularly for manufacturing a layer stack according to embodiments described herein.

The apparatus 500 can include at least three chamber portions 502A, 502B and 502C. At chamber portion 502C, one or more deposition sources 530 and optionally an etching station 430 can be provided as processing tools. A substrate 41, e.g. a flexible substrate, is provided on a first roll 764, e.g. having a winding shaft. The flexible substrate is unwound from the roll 764 as indicated by the substrate movement direction shown by arrow 108. A separation wall 701 is provided for separation of chamber portions 502A and 502B. The separation wall 701 can further be provided with gap sluices 140 for having the substrate 41 pass therethrough. A vacuum flange 112 provided between the chamber portions 502B and 502C can be provided with openings to take up at least some processing tools.

The substrate 41 is moved through the deposition areas provided at a coating drum 110 and corresponding to positions of the deposition sources 530. During operation, the coating drum 110 rotates around an axis such that the substrate 41 moves in the direction of arrow 108. According to some embodiments, the substrate 41 is guided via one, two or more rollers from the roll 764 to the coating drum 110 and from the coating drum 110 to the second roll 764', e.g. having a winding shaft, on which the substrate 41 is wound after processing thereof.

According to some embodiments, the deposition sources 530 can be configured for depositing the layers of the layer stack and the upper layer as described in embodiments of the present disclosure. As an example, at least one deposition source 530 can be adapted for deposition of the layer material having the refractive index of at least 1.9, at least one deposition source 530 can be adapted for deposition of the layer material having the refractive index of less than 1.7, and a deposition source 530 can be adapted for deposition of the layer material corresponding to the upper layer.

In some embodiments, a first deposition source can be configured for depositing the first layer, a second deposition source can be configured for depositing the second layer, a third deposition source can be configured for depositing the third layer, a fourth deposition source can be configured for depositing the fourth layer, and a fifth deposition source can be configured for depositing the upper layer.

In some implementations, the first chamber portion 502A is separated in an interleaf chamber portion unit 502A1 and a substrate chamber portion unit 502A2. Additionally, interleaf rolls 766/766' and interleaf rollers 105 can be provided as a modular element of the apparatus 500. The apparatus 500 can further include a pre-heating unit 194 to heat the flexible substrate. Further, additionally or alternatively a pre-treatment plasma source 192, e.g. an RF (radio frequency) plasma source can be provided to treat the substrate with a plasma prior to entering chamber portion 502C.

According to yet further embodiments, which can be combined with other embodiments described herein, optionally also an optical measurement unit 494 for evaluating the result of the substrate processing and/or one or more ionization units 492 for adapting the charge on the substrate can be provided.

According to some embodiments, the deposition material may be chosen according to the deposition process and the later application of the coated substrate. For instance, the deposition material of the deposition sources 630 may be silicon. As an example, oxide-, nitride- or carbide-layers, which can include such materials, can be deposited by providing the material from the source or by reactive deposition, i.e. the material from the source reacts with elements like oxygen, nitride, or carbon from a processing gas.

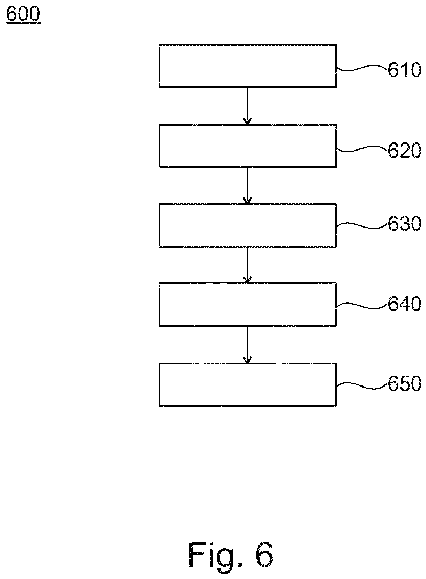

According to an aspect of the present disclosure and as shown in figure 6, a method 600 for manufacturing an anti-reflective and anti-viral protective shield and/or an anti-reflective and anti-viral visor for a face protection system is provided. The method may include alternately depositing a first layer material and a second layer material on a substrate to form a layer stack, particularly to form at least a first layer, a second layer, a third layer, and a fourth layer arranged in this order. The first layer material has a refractive index of at least 1.9 and the second layer material has a refractive index of less than 1.7. A first layer may be deposited for instance on a substrate (block 610). Then, a second layer may be deposited on or over the first layer (block 620). Afterwards, a third layer may be deposited on or over the second layer (block 630). Thereafter, a fourth layer may be deposited on or over the third layer (block 640). Further, the method 600 includes depositing a third material on or over the layer stack to form the upper layer (block 650).

Alternatively, the method 600 for manufacturing an anti-reflective and anti-viral protective shield and/or an anti-reflective and anti-viral visor for a face protection system includes alternately depositing a first layer material and a second layer material on a first surface side of a substrate to form a first layer stack, particularly to form at least a first layer, a second layer, a third layer, and a fourth layer arranged in this order.

In some embodiments, the method 600 may include alternately depositing a first layer material and a second layer material on a second surface side of the substrate to form a second layer stack, particularly to form at least a first layer, a second layer, a third layer, and a fourth layer arranged in this order. The first layer material has a refractive index of at least 1.9 and the second layer material has a refractive index of less than 1.7. The first surface side of the substrate is opposite the second side of the substrate.

In some embodiments, a first layer may be deposited for instance on or over a first surface side of a substrate and/or on or over a second surface side of a substrate. Then, a second layer may be deposited on or over the first layer. Afterwards, a third layer may be deposited on or over the second layer. Thereafter, a fourth layer may be deposited on or over the third layer. Further, the method 600 includes depositing a third material on or over the first layer stack and/or the second layer stack to form the upper layer. In some embodiments, the method 600 may include depositing a third material on or over a second surface side of a substrate to form an anti-viral layer.

Although in the present example of figure 5, five layers corresponding to the stack layer and the upper layer are arranged over each other, the present embodiments are not limited thereto. Any number of layers can be arranged, as it is for instance described above with reference to figures 1A to 1C, 2A, and 2B. Further, the method 600 for manufacturing an anti-reflective and anti-viral protective shield is similar to a method for manufacturing an anti-reflective and anti-viral protective visor for a face protection system.

While the foregoing is directed to embodiments of the disclosure, other and further embodiments of the disclosure may be devised without departing from the basic scope thereof, and the scope thereof is determined by the claims that follow.

## Claims

1. Anti-reflective and anti-viral protective shield, comprising:
a substrate;
a layer stack over the substrate, wherein the layer stack comprises at least a first layer, a second layer, a third layer, and a fourth layer arranged in this order,
wherein the first layer and the third layer have a refractive index of at least 1.9,
wherein the second layer and the fourth layer have a refractive index of less than 1.7; and
an upper layer over the layer stack,
wherein the upper layer comprises at least one material selected from the group consisting of TiO₂, ZnO, CuO, Ag-TiO₂, and combinations thereof.

2. Anti-reflective and anti-viral protective shield of claim 1, wherein the upper layer has a thickness equal to or smaller than 5 nm, particularly a thickness equal to or smaller than 3 nm, and more particularly a thickness equal to or smaller than 1 nm.

3. Anti-reflective and anti-viral protective shield of claims 1 or 2, wherein the layer stack further comprises one or more further layers over the fourth layer, particularly a fifth layer, or a fifth and a sixth layer.

4. Anti-reflective and anti-viral protective shield of one of claims 1 to 3, wherein the layers of the layer stack are directly disposed on each other.

5. Anti-reflective and anti-viral protective shield of one of claims 1 to 4, wherein odd numbered layers of the layer stack have the refractive index of at least 1.9, particularly of about 2.0.

6. Anti-reflective and anti-viral protective shield of one of claims 1 to 5, wherein the even numbered layers of the layer stack have a refractive index of less than 1.7, particularly a refractive index of less than 1.5, and more particularly of about 1.46.

7. Anti-reflective and anti-viral protective shield of one of claims 1 to 6, wherein odd numbered layers of the layer stack include at least one material selected from the group consisting of SiNx, NbOx, SiN, SiOxNy, AlOx, AlOxNy, TiOx TaOx, an organic material, particularly a polymer material, and combinations thereof and/or wherein even numbered layers of the layer stack include at least one material selected from the group consisting of SiOx, MgFx, SiOxNy, an organic material, particularly a polymer material, and combinations thereof.

8. Anti-reflective and anti-viral protective shield of one of claims 1 to 7, wherein the substrate comprises at least one material selected from the group consisting of polycarbonate, cellulose acetate propionate, polyvinyl chloride, polyacrylate and/or derivatives of polyacrylate, polymethacrylate and/or derivatives of polymethacrylate, polyethylene terephthalate glycol, polyethylene terephthalate, and combinations thereof.

9. Anti-reflective and anti-viral protective shield of one of claims 1 to 8, wherein the at least one material of the upper layer differs from the material of the uppermost layer of the layer stack on which the upper layer is directly disposed.

10. Anti-reflective and anti-viral protective shield of one of claims 1 to 9, wherein at least one of the layers of the layer stack has a thickness of more than 10 nm, particularly has a thickness of more than 20 nm, and more particularly has a thickness of more than 30 nm.

11. Anti-reflective and anti-viral protective shield of one of claims 1 to 10, wherein a light transmittance of the layer stack and/or a light transmittance of the layer stack and the upper layer is at least 85%, and particularly more than 90%.

12. Anti-reflective and anti-viral visor for a face protection system comprising an anti-reflective and anti-viral protective shield according to any of the preceding claims.

13. Method for manufacturing an anti-reflective and anti-viral protective shield and/or an anti-reflective and anti-viral visor for a face protection system, comprising:
alternately depositing a first layer material and a second layer material on a substrate to form a layer stack comprising at least four layers,
wherein the first layer material has a refractive index of at least 1.9,
wherein the second layer material has a refractive index of less than 1.7; and
depositing a third layer material on the layer stack to form an upper layer,
wherein the upper layer comprises at least one material selected from the group consisting of TiO₂, ZnO, CuO, Ag-TiO₂, and combinations thereof.

14. Method for manufacturing an anti-reflective and anti-viral protective shield and/or an anti-reflective and anti-viral visor for a face protection system of claim 13, wherein the upper layer has a thickness equal to or smaller than 5 nm, particularly a thickness equal to or smaller than 3 nm, and more particularly a thickness equal to or smaller than 1 nm.

15. Face protection system comprising the anti-reflective and anti-viral visor of claim 12 and a head connection unit.
